# EUROPEAN PATENT APPLICATION

(11) **EP 3 496 107 A1**
(43) Date of publication of application: **12.06.2019**
(21) Application number: 17206321.6
(22) Date of filing: 11.12.2017
(51) Int. Cl.: G16H 40/20

(54) **METHODOLOGY FOR THE OPTIMIZED MANAGEMENT OF RESOURCES IN THE PROVISION OF HEALTH SERVICES**

(71) Applicant: Commedia S.r.l., 73100 Lecce (IT)
(72) Inventor: DEL PRETE, Antonio, 73100 LECCE (IT); CAPALBO, Vincenzo, 73100 LECCE (IT); BENVENUTO, Fabrizio, 73100 LECCE (IT)
(74) Representative: Conversano, Gabriele

(57) **Abstract**

Method for health care service reservation management by a facility which has a plurality of human and equipment resources at disposal, to each one of said resources a list of types of services being associated which can be supplied by the resource as well as a calendar which plans the use in time during which the same is available, said calendar comprising a plurality of time intervals (timeslots) inside each work shift of said resource, the method comprising the following steps of:
a. acquisition of the user health care service request;
b. assigning the appointment for health care service supplying by at least one of said human and/or equipment resources to a calendar timeslot of said resource, which is free of previous appointments;
and being characterized in that
- to said request a property is associated which defines the request urgency category and, so, the time planning window within which the respective service has to be carried out for said request;
- to each one of said timeslots a property is associated which indicates the request urgency category for which the respective services can be supplied in said timeslot.

## Description

### Technical field and state of the art

Health-care services demand trend is definitely increasing, above all due to general population ageing. For this reason, an optimal management of both human and technological resources used in health-care field, is an always more important variable to be considered by health-care services facilities. An efficient management can lead to remarkable costs saving and to general improvement of services supplied in terms of user accessibility.

According to what known at the state of the art, the scheduling activity of medical staff daily working plan (and, where needed, of equipment resources) is provided manually and only after common sense rules. This fact implies a waste of time in scheduling activity, and generally, not so good solutions.

Vice versa, by using suitable support systems for planning and decision making it is possible to find out solutions which maximize the supplied service quality and which, as a consequence, reduce the dissatisfaction level both of clients and specialist doctors.

The resources capacity management is a very complex activity, which requires high competence. Its management aimed at reaching high efficiency levels could lead many facilities, as for example hospitals and health-care centers (whether public or private), to guarantee high level services with remarkable cost reductions.

The adoption of an efficient system for health-care service supply programming by a group of human and equipment resources becomes a complex activity. In order to guarantee an efficient use of the resources themselves, programming should have flexibility features besides adaptation capacity to cope with a certain number of present and future requests.

The choice of new methodological approaches could make health care services supply activity more efficient: from the assistance requests acquisition step to the evaluation of them, to the planning and programming of interventions and equipe, to the service supply.

Currently, in most cases, scheduling of appointments relating to health care services requests to both public and private facilities is still provided by the staff in charge without using any support system for decision making. When a health-care service request arrives, the scheduler selects manually a suitable time interval (TIMESLOT) on the calendar, which can be referred to a resource. The people who does this activity has to consider all the limits linked to the resources allocation process (capacity): the absence of a general view of all the decisional aspects linked to scheduling often leads to affect the whole activity, thus generating consequent difficulties in accessing to resources, and causing delays immediately felt by people requesting health care services.

In most cases, the person in charge to manage the calendar associated to resources is forced to determine the allocation of the resources capability at disposal in great advance, relying on his experience besides a forecast based on historical data, sometimes with collaboration of other people in charge. This kind of calendar appointments management leads surely to long waiting lists. Sometimes, as hospitals data show, even the increase in service offer can prove to be useless since lacking a suitable programming, the new availabilities can be saturated by patients requesting and receiving repeated controls as well as by cases of many users using the emergency room improperly. In fact, users go to the emergency room to avoid waiting lists in case of not urgent specialist visits, or to obtain prescriptions.

In many cases, the actual request of health care interventions does not correspond to allocated human and equipment resources capacity: paradoxically, the result is a scarcely efficient use of service supply capacity, and at the same time, long times for service supply, thus increasing the dissatisfaction of the patients involved.

A static allocation procedure of service requests to facilities (resources) can reduce strongly the efficiency of resources at facility disposal.

There is also another aspect to be considered, i.e. the demand variability. Currently, the person in charge of the activity tries to reduce some inefficiencies by manually correcting the calendar with the aim of adapting the resources allocation to the demand variability as best as possible. All of this requires another activity of calendar supervision with clear waste of time and energies, since possible corrections depend only on the supervisor experience: so, also a short absence or illness period of the supervisor leads to problems difficult to be overcome. Moreover, the adoption and the next learning step of a new person responsible for the calendar, would require an unacceptable time.

In addition to the just described problems it is to be added that from a careful analysis of literature about real cases relating to health care service supply it has emerged that the rule according to which the one, who makes the request first, is served firstly (FCFS - First Come First Serve) is unfair and often damages the one who has been in the utmost need.

Therefore, aim of the present invention is to provide a system for reservation programming, which is able:
a) to consider the capacity of the resources at disposal (service facilities);
b) to manage urgent requests optimally, by reserving a certain number of timeslots for requests to be dealt with within an assigned time window (planning window);
c) to allow extemporaneous urgent requests to be managed as well.

The invention reaches the prefixed aims since it provides a method for health care service reservation management by a facility which has a plurality of human and equipment resources at disposal, to each one of said resources a list of types of services being associated which can be supplied by the resource as well as a calendar which plans the use in time during which the same is available, said calendar comprising a plurality of time intervals (timeslots) inside each work shift of said resource, the method comprising the following steps of:
a. acquisition of the user health care service request;
b. assigning the appointment for health care service supplying by at least one of said human and/or equipment resources to a calendar timeslot of said resource, which is free of previous appointments;
and being characterized in that
- to said request a property is associated which defines the request urgency category and, so, the time planning window within which the respective service has to be carried out for said request;
- to each one of said timeslots a property is associated which indicates the request urgency category for which the respective services can be supplied in said timeslot.

These and other advantages will be highlighted by the detailed description in the following, in which it is described a decisional method for planning health care service supply, which can be implemented also on computer means and which is accessible also remotely, which allows a resources optimization management useful for health care service supply.

### 2 Description of the new optimization method

According to a preferred embodiment, the implementation of the method for health care service planning is provided with a first and a second graphical interface with the user, both being usable as a function of the user profile. The first one is dedicated to users who want to request a health care service supply, the second one is instead designed for operators managing resources (physicians and nurses besides equipment resources) who manage the service supply.

### 2.1 Reservation of a service and management mode

By means of the first interface the user can choose at least a service he needs from a list of services which can be supplied by the health facility.

In the following, the user is allowed to indicate the type of supply (in terms of urgency) which with the service has to be supplied.

As a way of example a categorization divided in four service urgency types can be used: urgent, short, deferred, programmed.

A different programming time window corresponds to each one of these user types. For example, a urgent request could relate to services to be supplied during the day, a short request to services to be supplied within three days and so on, with the programming time window increasing with the various urgency categories.

After introducing both data, and after processing the same according to what described in the following, the date of the appointment and the resources to be allocated are individuated, and it is possible to communicate to the user the result of the operation with the reservation details (date, time, place and possibly the resources involved).

After describing the procedure from the point of view of the user (the people who requests a health care service) it is possible to describe the procedure from the point of view of those who manage the service.

The resource who supplies the service (physician or nurse), after choosing from a list of services which can be supplied by the health care facility, the service of his/her own competence, can view the control panel of appointments, which shows the resources involved. According to a suitable graphical interface the control panel shows the database contents containing all data relating to past and future appointments.

The panel lists, in date ascending order, the fixed appointments, and by means of suitable filters, allows the operator to refine the research of the same. Among information viewed there are reported the duration intended for service supply as well as the real duration of supply if the user has been served yet.

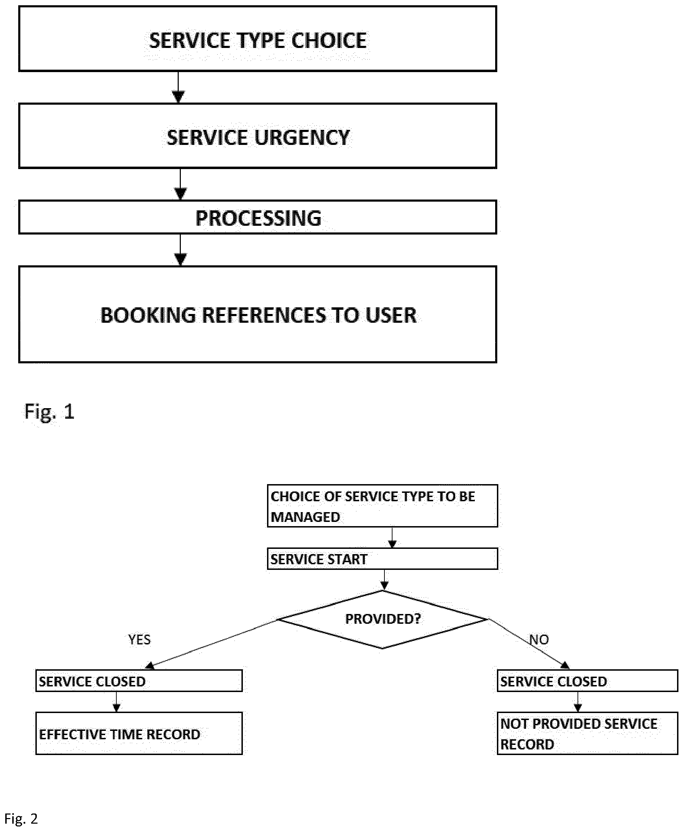

At the appointments fixed for the date of research the interface shows also two commands which allow the resource to signal to the system the start and the end of the service supply. By actuation of these commands the resource signals to the system if the service has been supplied or not, and the system records the real supply time. The flowchart is shown in figure 1.

After describing the interaction modes of the systems with subjects using it, it is possible to describe now, in detail, how the processing occurs, i.e. the passage during which an appointment is assigned to a request.

### Description of processing

Health care services are supplied thanks to a group of resources of a plurality of categories:
1. HUMAN RESOURCES
   - medical staff
   - nursing staff
2. EQUIPMENT RESOURCES (for example various diagnostic apparatuses)
3. STRUCTURAL RESOURCES (buildings and other facilities of the hospital).

The capacity available for each type of resource available will be given by the number of units referable to the same resource (for example the number of physician who can carry out ultrasound examinations) and by the time intervals of each of these resources, subdivided in shifts.

For each resource there are known the following properties:
- identifier (ID_RESOURCE) which characterizes it univocally in the system;
- resource type (medical staff, nursing staff, equipment or structural resource);
- description (resource name).

To each resource there are associated one or more services which can be supplied by the same, the type of which varies as a function of the resource skills. To each resource it is also associated a calendar which plans the use in time during which the same is available. Hence, to each shift of each operator there corresponds a set of available TIMESLOTS, during which the operator can be used for a service supply. So, the calendar of each operator will be subdivided in a TIMESLOT grid with variable dimension. To each one of these timeslots it will be associated a property which indicates the type of appointment (intended as different urgency level) which can be assigned to the resource in that time interval. In this manner, it will be possible to allocate the capacity of the resource required to a type of patient (for example URGENT, SHORT...).

Hence, for the whole health care facility it is possible to define a complete calendar where, for each day it is indicated a list of resources who can be used in that day, and for each one of these resources both the services which can be supplied and the type of each time interval (for urgent, short, deferred... requests) are known.

The complete calendar of the facility, defined in this manner, has to be used to assign an appointment to each request. A single request is characterized by a certain number of properties which are described in the following table:

**Table 1: Properties describing service request**

| REQUEST PROPERTIES | |
|---|---|
| Identifier | Univocal request identification code, associated to the medical prescription of the request |
| Tax identification number | Client tax identification number |
| Exempt | Flag (yes/no) to indicate the possible tax exemption |
| Ministerial code | Ministerial code relating to the service requested |
| Description | Health care service description |
| Path | If it is reported, it indicates the belonging to a PDT |
| Priority profile | Priority class defined by flags U, S, D, P |
| F/C | It indicates if the request is a first visit (F) or control (C) |
| Date | Service request date |

The request is forwarded by means of a medical prescription whose number identifies it univocally inside the system. The same request can be referred to:
- SINGLE service: i.e. the request for a control visit or a diagnostic service.
- MULTIPLE service: the request of a control visit associated to one or more diagnostic services. In this case, the various services identified by the same number of prescription have to be dealt with in the same day.

After the request acquisition step and the processing step of the same with appointment assignment, a resource supplies the requested service. From the point of view of the method execution, the following properties are assigned to each service:

**Table 2 - Properties describing health care service**

| SERVICE PROPERTIES | |
|---|---|
| Ministerial code | Ministerial code relating to the type of service supplied |
| Duration | Service time duration |
| Costs | Service tariff |
| Visit type | It describes the service type (class) (visit, equipment, visit + equipment) |
| Type of equipment resource | Type, if involved, of the equipment used in supplying the health care service |
| Nurse | Flag (0/1) indicating the need of a nurse or not |
| Description | Service description |

The capacity of resources at disposal is allocated to various types of services which can be supplied (classes). The allocation process has to observe limits both of medical nature (relating to the skill levels needed to carry out a particular service) and objective relating to scheduling efficacy (reserving a certain number of timeslots for services with a higher urgency feature).

This double result can be obtained by modeling the allocation by using timeslot-type specification (TTS) or specific of the type for each timeslot. A timeslot-type specifies which service request type can be scheduled in each timeslot of each resource. In this manner, it is possible to define which percentage of each resource capacity is allocated for requests with each urgency profile: moreover, TTS can be varied according to the needs thus making the allocation much more dynamic.

**Table 3 - definition of TimeSlot Type Specification**

| TTS | Type of allowed requests |
|---|---|
| TTUrgent | Services with U profile |
| TTShort | Services with S profile |
| TTDeferred | Services with D profile |
| TTProgrammed | Services with P profile |

In order to overcome the problems generated by a manual management of appointments calendar for service requests with different urgency profiles, the capacity to allocate by each resource and by the different urgency profiles is virtually subdivided during the scheduling step.

In detail a certain number of timeslots is specifically reserved for requests which will be presented on a determined date and which have to be dealt with in an assigned PLANWIN (i.e. within a determined time interval starting from the request).

The number of timeslots which is reserved for the different urgency profiles (PLANWIN) and the date of request entry (which defines the dimension of reserve) is determined on the basis of the forecast of the expected number of services requested, with a little additional capacity surplus.

Once it is determined which timeslots are reserved to them, according to first come first serve (FCFS) policy the requests of URGENT type are scheduled to timeslot, whether reserved or not, but corresponding both to the day and the PLANWIN of the request to be managed.

In order to make the variability more flexible and to reduce its usage at minimum, a violation of the reservation policy is allowed if the request is not scheduled on time.

In this case, if in the day of request there is no timeslot available corresponding to the urgency level of the client requesting the service, the request is scheduled during the day, still within the acceptable time interval (PLANWIN) which has the most timeslots available, without using those provided by the matrix of reserves.

In order to overcome the difficulties generated by the need to manage requests with different urgency profiles with the same resources, the proposed method tries to define a fair compromise between two fundamental aspects in order to guarantee the allocation efficacy:
- to schedule requests by exploiting nearer and immediately available timeslots on scheduling calendar in order not to waste capacity;
- to save or keep available timeslots to manage requests of services with a higher urgency profile.

Currently, the persons in charge who carry out scheduling activities cannot have a general view of the problem, which allows them to manage these operations efficiently. Therefore one risks:
- to schedule services with a lower urgency profile too earlier than those with a higher urgency profile;
- to waste too much capacity of a resource.

With reference now to the features of the calendar subdivision method, it is specified that timeslots in which the calendar is subdivided will have the dimension of a multiple of a time unit (tu). As a way of example, a time unit is 15 or 30 minutes. Parameters m (number of resources) and tu (time unit duration) are constant for the scheduling time, while other parameters, which are listed in the following, can vary dynamically.

**Table 4 - Definition of calendar parameters**

| CALENDAR PARAMETERS | |
|---|---|
| m | Number of operators at disposal |
| O_{j,d} | Beginning of working day of operator j in date d |
| C_{j,d} | End of working day of operator j in date d |
| tu | Unit of measurement of elementary timeslot |
| TTS | TimeSlot Type Specification |

In the context of health care service supply, the capacity allocation by means of TTS on calendar associated to each resource can be considered from the point of view of the time-frame it is referred to:
1. long term (months): allocation is determined on the basis of historical data relating to previous periods of time and relating to the expected number of service requests or on the basis of decisional policy of the persons in charge of the supply facilities:
2. medium term (weeks): in this case, corrections to previous allocation can be carried out to deal with not ordinary, foreseeable events and whose dimension is known: absence periods, additional working loads. These corrections could relate also to working hours of operators, still under contract conditions;
3. short term (days): in this last case corrections to allocations are carried out daily; these adjustments are based on the current number of requests and on the one expected in future.

The method proposed refers to the methodological approach of adaptive type for short term adjustments, and the various steps are described in the following section. The approach defined as short-term adaptive is developed through two main steps:
- a scheduling step (flexible reservation) of service requests by means of a heuristics based on daily reserving some timeslots (see relating section) and
- a following step to carry out corrections which allow the not allocated capacity to be redistributed among the various request classes. "Flexible reservation" scheduling step - description of methodological logic

The aim of this first step is to provide a scheduling of different request classes, such for example the URGENT request class and the SHORT type one.

In order to guarantee the scheduling of these request types in the time intended by respective planning time window, the capacity of allocated resources has to be sufficiently great. The requests of service refer to different TTS:
- URGENT services with (0.1), (0.2) or (0.3) PLANWIN use TTurgent type timeslots;
- SHORT services with (0.10) PLANWIN use TTSHORT type timeslots.

PLANWINs are defined on the basis of the urgency associated to the various requests. On the basis of the urgency level patients are subdivided in groups to which respective suitably dimensioned PLANWINs correspond.

In fact, in particular (0.1), (0.2) and (0.3) PLANWINs correspond to patients with urgent requests. In other words, services with a planwin flag of this type are treated with a urgency profile since they can be carried out in 1 day (0.1), 2 days (0.2) and 3 days (0.3) PLANWINs, but not later. At the same manner, patients with "short" service requests will be characterized and so recognized as such by the system thanks to the fact that PLANWINs (0.10) will be associated, i.e. they have to be carried out at the latest within 10 days.

As yet said, the capacity to be allocated for urgencies is virtually subdivided during the scheduling step. Practically, a certain number of timeslots is specifically reserved for requests which have to be dealt with in a certain PLANWIN (the whole for each day of request entry).

Table 6 defines the matrix of reserves dedicated for example to requests of URGENT type, R(Tturgent)planwinreqd, for each PLANWIN and day of request entry reqd (referred to the current day d = 0). In similar manner, a matrix of reserves is defined for each type of request requiring a high urgency profile.

**Table 6 - Matrix for positioning timeslots reserved for TTURGENT type**

| Timeslot reserved inside TTS of URGENT type | | | |
|---|---|---|---|
| | Day of service request (reqd) | | |
| PLANWIN | 0 | 1 | reqd |
| (0.1) | R (TTurgent) ^{(0.1)}_{reqd=0} | R (TTurgent) ^{(0.1)}_{reqd=1} | R (TTurgent) ^{(0.1)}_{reqd} |
| (0.2) | R (TTurgent) ^{(0.2)}_{reqd=0} | R (TTurgent) ^{(0.2)}_{reqd=1} | R (TTurgent) ^{(0.2)}_{reqd} |
| (0.3) | R(TTurgent) ^{(0.3)}_{read=0} | R(TTurgent) ^{(0.3)}_{read=1} | R (TTurgent) ^{(0.3)}_{reqd} |

In the definition step of which timeslots have to be reserved, a heuristic approach defined as "Flexible Reservation" is used to position reserves in TTS on calendar. The reserved timeslots are positioned on the last day of PLANWIN, in this manner the variability of their usage is reduced at minimum.

**Table 7 - positioning timeslots reserved for TTURGENT type**

| today d = 0 | d = 1 | d = 2 | d = 3 | d = 4 |
|---|---|---|---|---|
| | | | R (TTurgent) ^{(0.3)}_{reqd=0} | R (TTurgent) ^{(0.3)}_{reqd=1} |
| | | R (TTurgent) ^{(0.2)}_{reqd=0} | R (TTurgent) ^{(0.2)}_{reqd=1} | R (TTurgent) ^{(0.2)}_{reqd=2} |
| | R(TTurgent) ^{(0.1)}_{reqd=0} | R (TTurgent) ^{(0.1)}_{reqd=1} | R(TTurgent) ^{(0.1)}_{reqd=2} | R(TTurgent) ^{(0.1)}_{reqd=3} |

### "Adjusting capacity" step

The step following to the starting scheduling one controls if timeslots initially reserved, have been really used or not. In fact, those not used by a particular type of request could be made available for other request classes; in this manner, a management of resources capacity allocation derives with dynamic elements and able to be adapted to the following requests of health care services.

Practically, at the beginning of the current day which will be indicated with notation d = 0, the capacity available is moved among the various urgency profiles of requests (TTS), in particular in days with PLANWINs overlapping.

An exchange among for example TTUrgent and TTShort classes in the current day d = 0 is not needed, since the first step of approach (the scheduling one) does not provide to reserve timeslot in the current day. This measure allows a person in charge to correct the scheduling done at least a day in advance; in this manner it is possible to carry out the re-allocation operation of timeslots among the various urgency profiles only once a day without being forced to carry out such corrections continuously.

The aim reached with the second step of the proposed approach is to re-allocate, with simple adjustments, the capacity which can be referred to the resources available, in such a manner that all the timeslots can be always reserved in the context of the capacity intended for that particular type of request. The number of timeslot which is exchanged among the different classes depends on the number of reserves.

In this manner, each timeslot, which is no more needed for the reserve step, can be changed in a timeslot re-usable by another category of request.

## Claims

1. Method for health care service reservation management by a facility which has a plurality of human and equipment resources at disposal, to each one of said resources a list of types of services being associated which can be supplied by the resource as well as a calendar which plans the use in time during which the same is available, said calendar comprising a plurality of time intervals (timeslots) inside each work shift of said resource, the method comprising the following steps of:
a. acquisition of the user health care service request;
b. assigning the appointment for health care service supplying by at least one of said human and/or equipment resources to a calendar timeslot of said resource, which is free of previous appointments;
and being **characterized in that**
- to said request a property is associated which defines the request urgency category and, so, the time planning window within which the respective service has to be carried out for said request;
- to each one of said timeslots a property is associated which indicates the request urgency category for which the respective services can be supplied in said timeslot.

2. Method according to claim 1, **characterized in that** said step a. is preceded by a scheduling step in which a capacity of each resource to be allocated for each urgency profile is assigned on the basis of the number expected of services requested for each urgency category and for each day of request entry.

3. Method according to claim 1 or 2, **characterized in that** during each scheduling time the duration of timeslots and the resources available are constant, **in that** during said scheduling step to each timeslot a urgency category is assigned, and **in that**, periodically, after said scheduling step an adjusting capacity step is carried out in which the timeslots not used for a particular category of requests are made available for other request categories.

4. Method according to claim 1 or 2, **characterized in that** said adjusting capacity step is carried out once a day.
